# EUROPEAN PATENT APPLICATION

(11) **EP 4 644 533 A1**
(43) Date of publication of application: **05.11.2025**
(21) Application number: 23910936.6
(22) Date of filing: 29.12.2023
(51) Int. Cl.: C12N 1/21, C12N 15/63, C12N 15/70, C12N 15/67, C12P 19/04

(54) **CONSTRUCTION AND USE OF ENGINEERING BACTERIUM THAT PRODUCES COLANIC ACID WITH HIGH YIELD**

(30) Priority: 30.12.2022 WO PCT/CN2022/143748; 23.08.2023 WO PCT/CN2023/114524
(71) Applicant: Shenzhen Pam2L Biotechnologies Co., Ltd., Shenzhen, Guangdong 518132 (CN)
(72) Inventor: JIN, Xuerong, Shenzhen, Guangdong 518132 (CN); CUI, Junfeng, Shenzhen, Guangdong 518132 (CN); LI, Jiaming, Shenzhen, Guangdong 518132 (CN)
(74) Representative: Barrow, Nicholas Martin
(86) International application number: PCT/CN2023/143258
(87) International publication number: WO 2024/141022

(57) **Abstract**

The present disclosure provides a recombinant engineered strain for efficiently producing colanic acid (CA) and uses thereof. By editing key genes for CA synthesis, that is, rcsA and rcsB, galU, galE, ugD, manB, manC, gmd and fcl genes, acetic acid pathway genes, cofactor regeneration related genes and CA synthesis cluster relatd genes, and by means of overexpression in two modes, i.e., plasmid and genomic integration, the recombinant engineered strain that produces CA with a high yield is obtained. The provided recombinant engineered strain provides a basis for large-scale production..

## Description

### CROSS REFERENCE TO RELATED APPLICATIONS

This application claims the benefit and priority of PCT International Application No. PCT/CN2022/143748, filed on Dec. 30, 2022, and PCT International Application No. PCT/CN2023/114524, filed on Aug. 23, 2023, the entire contents of which are incorporated herein by reference.

### TECHNICAL FIELD

The present invention belongs to the field of genetic engineering and fermentation engineering, and specifically relates to the construction and use of an engineered strain with high yield of colanic acid.

### BACKGROUND

Colanic acid (CA) is a bacterial exopolysaccharide, which is produced by most *Escherichia coli* (*E. coli*) strains and other species of the *Enterobacteriaceae* family, and is a polysaccharide synthesized by bacteria in order to adapt to environmental changes and improve the self-survival probability during life activities. CA has a large molecular weight and is loosely wrapped on the surface of the bacterium, so that the bacterium shows a mucus state, preventing the cells from losing water, protecting the cells and resisting harmful substances. Under environmental conditions that are not conducive to growth, such as dryness, low pressure, and low pH, mucilaginous strains have greater survivability than wild strains. In 2017, Han *et al.* reported that the life of *C. elegans* can be significantly prolonged by feeding purified CA or feeding *E. coli* that can secrete CA. In addition, CA, as a unique active biopolymer, has special biological properties and physiological parameters, and has a wide application prospect. For example, CA is a natural hydrogel with excellent water-replenishing ability and soft texture due to its porous cellulose structure and a large number of hydrophilic groups on the surface of the colloid, making it a promising candidate product for future cosmetics and healthcare markets.

In the *in vivo* study using zebrafish as a model, it is found that polysaccharides or oligosaccharides containing fucose have clear anti-inflammation functions. In addition, studies have shown that fucose molecules, or polysaccharides or oligosaccharides containing fucose can more easily penetrate into the dermis, increase the skin thickness and promote finer collagen structure, so that fucose as well as polysaccharides or oligosaccharides rich in fucose can slow down skin aging. CA is composed of D-glucose, L-fucose, D-galactose, D-glucuronic acid, and non-stoichiometrically modified O-acetyl and pyruvic acid on the side chains, wherein the content of L-fucose is up to about 30%, which means that CA will have great application potential in terms of anti-inflammation, immunity enhancing and anti-aging.

The colanic acid that is synthesized by *E. coli* under certain conditions has a large molecular weight, which is about 8~10 million Da, and such large molecular weight polysaccharides have certain applications in the fields of cosmetics, health food and the like. At present, colanic acid is mainly synthesized in *E. coli,* and studies show that when relevant genes of *E. coli* are knocked out and/or overexpressed, the synthesis of colanic acid can be achieved under certain culture conditions. For example, SUN Junsong et al. (Patent CN109439708A) achieved high yield of CA by intracellularly transfecting a plasmid pBhya-CAB into an acid-resistant *E. coli,* and the yield reached 10.22 g/L; however, this strain needs to be cultured under a low pH condition, and simultaneously produces PHB as a by-product, which is detrimental to subsequent product separation. WANG Xiaoyuan et al. (Patent CN113755515A) knocked out genes related to lipopolysaccharide synthesis in *E. coli* and overexpressed two genes in the precursor synthesis pathway, finally achieved synthesis of CA at 19.79 g/L; however, this strain has a long cultivation cycle, requiring 72 hours to complete a fermentation batch, resulting in low production efficiency.

The present invention aims to engineer strains through a series of synthetic biological technical means to prepare several engineered strains that can efficiently produce CA, and provide a basis for large-scale production of CA.

### SUMMARY

In one aspect, the present disclosure provides a recombinant engineered strain, wherein in the recombinant engineered strain,
a) lipopolysaccharide core polysaccharide synthesis gene cluster waaL, waaU, waaZ, waaY, waaR, waaO, waaB, waaP, waaG, and waaQ in the genome have been knocked out; and Lon protein coding gene lon and HNS regulatory protein coding gene hns have been knocked out;
b) DNA binding transcription activator RcsA coding gene rcsA and DNA binding transcription activator RcsB coding gene rcsB have been overexpressed; and
c) UTP-1-glucose phosphate uridylyltransferase coding gene galU, UDP-glucose-4-isomerase coding gene galE, UDP-glucose-6-dehydrogenase coding gene ugD, phosphomannanase coding gene manB, mannose-1-phosphate guanylyl transferase manC, GDP-mannose-4,6-dehydratase coding gene gmd, and GDP-L-fucose synthase coding gene fcl have been overexpressed.

In some specific embodiments, the genes overexpressed in b) and c) are derived from any one of *E. coli, Bacillus subtilis, Saccharomyces cerevisiae, Pichia pastoris, Kluyveromyces lactis,* or *Streptomycetaceae,* preferably *E. coli.*

Those skilled in the art should appreciate that in different engineered strains, the specific sequences of the above genes may be homologous rather than completely identical, and it may be expected that the functions of the same gene in different strains are highly similar, therefore the specific gene sequences do not limit the scope of the present disclosure.

In some specific embodiments, in the recombinant engineered strain, a Ptac promoter is used to enhance the expression of rcsA, rcsB, galU, galE, ugD, manB, manC, gmd and fcl genes.

In some specific embodiments, in the recombinant engineered strain, a Ptac promoter P2 is used to enhance the expression of rcsA and rcsB, a Ptac promoter P3 is used to enhance the expression of galU, galE, and ugD, a Ptac promoter P4 is used to enhance the expression of manB and manC, a Ptac promoter P1 is used to enhance the expression of gmd and fcl, wherein the sequence of said P1 comprises SEQ ID NO: 1, the sequence of said P2 comprises SEQ ID NO: 2, the sequence of said P3 comprises SEQ ID NO: 3, the sequence of said P4 comprises SEQ ID NO: 4.

In some specific embodiments, the sequence of said P1 is as shown in SEQ ID NO: 1, the sequence of said P2 is as shown in SEQ ID NO: 2, the sequence of sad P3 is as shown in SEQ ID NO: 3, the sequence of said P4 is as shown in SEQ ID NO: 4.

In some specific embodiments, the 5' ends of the rcsA, rcsB, galU, galE, ugD, manB, manC, gmd, and fcl genes are linked to ribosome binding site RBS sequence.

In some specific embodiments, the ribosome binding site RBS sequence comprises a sequence selected from the group consisting of SEQ ID NOs: 5-31.

In some specific embodiments, the 5' end of rcsA is linked to a ribosome binding site RBS sequence R_{H1}, the 5' end of rcsB is linked to a ribosome binding site RBS sequence R_{H2}, the 5' end of galU is linked to a ribosome binding site RBS sequence R_{L3}, the 5' end of galE is linked to a ribosome binding site RBS sequence R_{L4}, the 5' end of ugD is linked to a ribosome binding site RBS sequence R_{L5}, the 5' end of manB is linked to a ribosome binding site RBS sequence R_{M6}, the 5' end of manC is linked to a ribosome binding site RBS sequence R_{M7}, the 5' end of gmd is linked to a ribosome binding site RBS sequence R_{L8}, the 5' end of fcl is linked to a ribosome binding site RBS sequence R_{L9}; wherein the R_{H1} sequence comprises SEQ ID NO: 5; the R_{H2} sequence comprises SEQ ID NO: 6; the R_{L3} sequence comprises SEQ ID NO: 25; the R_{L4} sequence comprises SEQ ID NO: 26; the R_{L5} sequence comprises SEQ ID NO: 27; the R_{M6} sequence comprises SEQ ID NO: 19; the R_{M7} sequence comprises SEQ ID NO: 20; the R_{L8} sequence comprises SEQ ID NO: 30; the R_{L9} sequence comprises SEQ ID NO: 31.

In some specific embodiments, the R_{H1} sequence is as shown in SEQ ID NO: 5; the R_{H2} sequence is as shown in SEQ ID NO: 6; the R_{L3} sequence is as shown in SEQ ID NO: 25; the R_{L4} sequence is as shown in SEQ ID NO: 26; the R_{L5} sequence is as shown in SEQ ID NO: 27; the R_{M6} sequence is as shown in SEQ ID NO: 19; the R_{M7} sequence is as shown in SEQ ID NO: 20; the R_{L8} sequence is as shown in SEQ ID NO: 30; the R_{L9} sequence is as shown in SEQ ID NO: 31.

In the present disclosure, the recombinant engineered strain is selected from any one of the *Enterobacteriaceae* family, more preferably from *Escherichia coli* BL21 (DE3), JM109, Nissle 1917 (EcN), BW23110 or MG1655.

In a second aspect, the present disclosure provides a method for constructing an engineered strain that produces colanic acid, comprising:
a) knocking out lipopolysaccharide core polysaccharide synthesis gene cluster waaL, waaU, waaZ, waaY, waaR, waaO, waaB, waaS, waaP, waaG, waaQ in the genome, and knocking out Lon protein coding gene lon and HNS regulatory protein coding gene hns;
b) overexpressing DNA binding transcription activator RcsA coding gene rcsA and DNA binding transcription activator RcsB coding gene rcsB;
c) overexpressing galU, galE, ugD, manB, manC, gmd and fcl genes.

In some specific embodiments, the genes overexpressed in b) and c) are derived from any one of *E. coli, Bacillus subtilis, Saccharomyces cerevisiae, Pichia pastoris, Kluyveromyces lactis,* or *Streptomycetaceae,* preferably *E. coli.*

In some specific embodiments, in the above method, in the recombinant engineered strain, a Ptac promoter P2 is used to enhance the expression of rcsA and rcsB, a Ptac promoter P3 is used to enhance the expression of galU, galE, and ugD, a Ptac promoter P4 is used to enhance the expression of manB and manC, a Ptac promoter P1 is used to enhance the expression of gmd and fcl, wherein the sequence of said P1 comprises SEQ ID NO: 1, the sequence of said P2 comprises SEQ ID NO: 2, the sequence of said P3 comprises SEQ ID NO: 3, the sequence of said P4 comprises SEQ ID NO: 4. Preferably, in a specific embodiment, the sequence of said P1 is as shown in SEQ ID NO: 1, the sequence of said P2 is as shown in SEQ ID NO: 2, the sequence of said P3 is as shown in SEQ ID NO: 3, and the sequence of said P4 is as shown in SEQ ID NO: 4.

In some specific embodiments, in the above method, the 5' ends of the rcsA, rcsB, galU, galE, ugD, manB, manC, gmd, and fcl genes are linked to ribosome binding site RBS sequence.

In some specific embodiments, in the above method, the ribosome binding site RBS sequence comprises a sequence selected from the group consisting of SEQ ID NOs: 5-31.

In some specific embodiments, in the above method, the 5' end of rcsA is linked to a ribosome binding site RBS sequence R_{H1}, the 5' end of rcsB is linked to a ribosome binding site RBS sequence R_{H2}, the 5' end of galU is linked to a ribosome binding site RBS sequence R_{L3}, the 5' end of galE is linked to a ribosome binding site RBS sequence R_{L4}, the 5' end of ugD is linked to a ribosome binding site RBS sequence R_{L5}, the 5' end of manB is linked to a ribosome binding site RBS sequence R_{M6}, the 5' end of manC is linked to a ribosome binding site RBS sequence R_{M7}, the 5' end of gmd is linked to a ribosome binding site RBS sequence R_{L8}, the 5' end of fcl is linked to a ribosome binding site RBS sequence R_{L9}; wherein the R_{H1} sequence comprises SEQ ID NO: 5; the R_{H2} sequence comprises SEQ ID NO: 6; the R_{L3} sequence comprises SEQ ID NO: 25; the R_{L4} sequence comprises SEQ ID NO: 26; the R_{L5} sequence comprises SEQ ID NO: 27; the R_{M6} sequence comprises SEQ ID NO: 19; the R_{M7} sequence comprises SEQ ID NO: 20; the R_{L8} sequence comprises SEQ ID NO: 30; the R_{L9} sequence comprises SEQ ID NO: 31. Preferably, in a specific embodiment, the R_{H1} sequence is as shown in SEQ ID NO: 5; the R_{H2} sequence is as shown in SEQ ID NO: 6; the R_{L3} sequence is as shown in SEQ ID NO: 25; the R_{L4} sequence is as shown in SEQ ID NO: 26; the R_{L5} sequence is as shown in SEQ ID NO: 27; the R_{M6} sequence is as shown in SEQ ID NO: 19; the R_{M7} sequence is as shown in SEQ ID NO: 20; the R_{L8} sequence is as shown in SEQ ID NO: 30; the R_{L9} sequence is as shown in SEQ ID NO: 31.

In some specific embodiments, in the above method, the engineered strain is selected from any one of the *Enterobacteriaceae* family, more preferably from *Escherichia coli* BL21 (DE3), JM109, Nissle 1917 (EcN), BW23110 or MG1655.

In some specific embodiments, the engineered strain uses any one of the *Enterobacteriaceae* family as a host cell, and uses a pET series vector, pRSFDuet-1, pACYDuet-1, pCDFDuet1 and related modified plasmids as expression vectors to overexpress genes.

In another aspect, the present disclosure provides a use of the recombinant engineered strain according to any one of the foregoing in the preparation of a product for producing colanic acid.

In another aspect, the present disclosure provides a recombinant engineered strain, wherein in the recombinant engineered strain,
a) lipopolysaccharide core polysaccharide synthesis gene cluster waaL, waaU, waaZ, waaY, waaR, waaO, waaB, waaP, waaG, and waaQ in the genome have been knocked out; and Lon protein coding gene lon and HNS regulatory protein coding gene hns have been knocked out;
b) acetic acid synthesis pathway gene poxB has been knocked out;
c) DNA binding transcription activator coding gene rcsA and DNA binding transcription activator coding gene rcsB have been overexpressed;
d) isocitrate dehydrogenase gene icd related to NADPH regeneration and/or pyridine nucleotide transhydrogenase coding gene pntAB have been overexpressed; guanosine kinase coding gene gsk related to GTP regeneration has been integrated and expressed;
e) UDP-1-glucose phosphate uridylyltransferase coding gene galU has been overexpressed;
f) UDP-glucose-1-phosphotransferase coding gene wcaJ and/or colanic acid polymerase coding gene wcaD related to colanic acid synthesis cluster have been overexpressed.

In some specific embodiments, the genes overexpressed in c) to f) are derived from any one of *E. coli, Bacillus subtilis, Saccharomyces cerevisiae, Pichia pastoris, Kluyveromyces lactis,* or *Streptomycetaceae,* preferably *E. coli.*

In another aspect, the present disclosure provides a method for constructing a colanic acid recombinant engineered strain, comprising:
a) knocking out lipopolysaccharide core polysaccharide synthesis gene cluster waaL, waaU, waaZ, waaY, waaR, waaO, waaB, waaP, waaG, and waaQ in the genome, and knocking out Lon protein coding gene lon and HNS regulatory protein coding gene hns;
b) knocking out acetic acid synthesis pathway gene poxB;
c) overexpressing DNA binding transcription activator coding gene rcsA and DNA binding transcription activator coding gene rcsB;
d) overexpressing isocitrate dehydrogenase gene icd related to NADPH regeneration and/or pyridine nucleotide transhydrogenase coding gene pntAB; integrating and expressing guanosine kinase coding gene gsk related to GTP regeneration;
e) overexpressing UDP-1-glucose phosphate uridylyltransferase coding gene galU;
f) overexpressing UDP-glucose-1-phosphotransferase coding gene wcaJ and/or colanic acid polymerase coding gene wcaD related to colanic acid synthesis cluster.

In some specific embodiments, the genes overexpressed in c) to f) are derived from any one of *E. coli, Bacillus subtilis, Saccharomyces cerevisiae, Pichia pastoris, Kluyveromyces lactis,* or *Streptomycetaceae,* preferably *E. coli.*

### BRIEF DESCRIPTION OF THE DRAWINGS

The invention will be more fully understood with reference to the following drawings.
**FIG. 1** shows a pTarget-waaF plasmid map.
**FIG. 2** shows a comparison before and after waaF and LQ knockout.
**FIG. 3** shows the yield corresponding to the lipopolysaccharide module knockout strain.
**FIG. 4** shows the effect of RcsCDB module-related gene knockout on CA yield and OD₆₀₀.
**FIG. 5** shows RcsCDB module-related plasmid construction.
**FIG. 6** shows single-gene enhancement of the precursor synthesis pathway.
**FIG. 7** shows dual-gene enhancement of the precursor synthesis pathway.
**FIG. 8** shows multiple genes enhancement of the precursor synthesis pathway.
**FIG. 9** shows the effect of gene arrangement order on yield.
**FIG. 10** shows the effect of promoter optimization on yield.
**FIG. 11** shows the enhanced expression frame corresponding to the engineered strain Δ3AB-Pc.
**FIG. 12** shows the relative fluorescence intensities of different RBSs.
**FIG. 13** shows the CA yield after optimization of promoter and RBS combinations.
**FIG. 14** shows the yield and pH change corresponding to the engineered strain Δ4.
**FIG. 15** shows the yield and OD600 corresponding to the engineered strain with integration and expression of rcsA and rcsB.
**FIG. 16** shows the yield and OD600 of strains with enhanced cofactor regeneration-related genes.
**FIG. 17** shows the yield and OD600 of strains with enhanced colanic acid synthesis cluster-related genes.
**FIG. 18** shows the yield corresponding to the optimal strains in a 5-L fermentation tank.

### DETAILED DESCRIPTION

Various features and aspects of the present invention are discussed in detail below.

The technical solution mainly includes three parts. The first and second parts are a first technical route for the construction of high-yield strains. The first part is a modular engineering strategy to achive CA yield improvement, which involves a lipopolysaccharide synthesis module, a RcsCDB phosphotransfer module, and a precursor synthesis pathway module. By performing corresponding knockout or overexpression on these modules, the metabolic flow direction and metabolic flow strength are changed, so that the yield is greatly improved. The second part is to screen and optimize components and to balance metabolic flow to improve CA yield. Promoter optimization, ribosome binding site (RBS) optimization as well as promoter and RBS combination to balance metabolism are involved. The third part is to investigate the CA synthesis related pathway by genomic integration, wherein the acetic acid synthesis pathway is knocked out, the RcsCDB phosphotransfer module is integrated and expressed, the cofactor regeneration pathway gene is overexpressed, and the CA synthesis cluster related gene is overexpressed. The starting strain may be selected from any one of the *Enterobacteriaceae* family, more preferably from *Escherichia coli* BL21 (DE3), JM109, Nissle 1917 (EcN), BW23110 or MG1655.

Implementations of overexpression described in the present disclosure include plasmid overexpression, or overexpression of genomic integration. It should be understood by those skilled in the art that, as long as the overexpression of the target gene is achieved in the strain, it is an implementation of overexpression.

### 1. Modular Engineering Strategy to Improve CA Yield

### 1.1 Lipopolysaccharide Module Knockout

Lipopolysaccharide is a main component of the outer membrane of most Gram-negative strains, the synthesis of lipopolysaccharide involves the consumption of precursors such as glucose (glu) and galactose (gal), and these precursors are also common raw materials for synthesizing CA polysaccharide, therefore the metabolic flow can be shifted to the synthesis of CA polysaccharide by knocking out the lipopolysaccharide synthesis pathway, thereby improving the yield. Relevant enzymes involved in the core sugar synthesis in lipopolysaccharides are encoded and synthesized by 15 genes (waaD, waaF, waaC, waaL, waaU, waaZ, waaY, waaR, waaO, waaB, waaS, waaP, waaG, waaQ, waaA) in the waa gene cluster. In the present disclosure, three ways of knockout are respectively performed: knocking out waaF, LQ (waaL, waaU, waaZ, waaY, waaR, waaO, waaB, waaaS, waaP, waaG, waaQ), LG (waaL, waaU, waaZ, waaY, waaR, waaO, waaB, waaaS, waaP, waaG), respectively, to obtain three recombinant strains ΔwaaF, ΔLG, and ΔLQ; and performing verification analysis on the three recombinant strains, respectively. The results show that all three strains synthesized CA, with the ΔLQ knockout showing a more significant effect.

### 1.2 RcsCDB Phosphotransfer Module

Based on the above ΔLQ, the Ion or hns genes were further knocked out, respectively, to obtain the engineered strains ΔLQΔIon and ΔLQΔhns. It is found that the CA yield of these two strains is significantly improved, and hns is further knocked out based on ΔLQΔlon to obtain a triple knockout strain ΔLQΔlonΔhns, which is analyzed and turns out that the yield is further improved, and it is the strain with the highest yield described above, and this strain is named as Δ3. Then, rcsA, rcsB, and rcsA+rcsB were further overexpressed based on the strain, wherein the optimal engineered strain is an rcsA+rcsB enhanced strain, named Δ3AB.

### 1.3 Precursor Synthesis Module

The genes related to the precursor synthesis pathway are pgi, pgm, galU, galE, ugD, manA (pmi), manB (cpsG, rfbK), manC (cpsB, mni, rfbM), gmd, fcl (wcaG, yefB). First, single-gene overexpression was performed on the precursor pathway genes, respectively, to obtain a series of single-gene expression-enhanced strains with different degrees of improvement in CA yield. Then, dual-gene enhancements pgm-galU (M-U), pgm-galE (M-E), pgm-ugD (M-D), galU-galE (U-E), galU-ugD (U-D), galE-ugD (E-D), manA-manB (A-B), manB-manC (B-C), manB-gmd (B-G), manB-fcl (B-F), manC-gmd (C-G), gmd-fcl (G-F), galU-manB (U-B), galU-manC (U-C), galU-gmd (U-G), and galU-fcl (U-F) were respectively performed, and different engineered strains obtained by dual-gene combination enhancement show varying degrees of increase in CA yield. It is easily conceivable that other dual-gene combinations may also have different degrees of increase in CA yield, such as pgm-manA (M-A), pgm-manB (M-B), pgm-manC (M-C), pgm-gmd (M-G), pgm-fcl (M-F), galU-manA (U-A), galU-manB (U-B), galU-manC (U-C), galU-gmd (U-G), galU-fcl (U-F), galE-manA (E-A), galE-manB (E-B), galE-manC (E-C), galE-gmd (E-G), galE-fcl (E-F), ugD-manA (D-A), ugD-manB (D-B), ugD-manC (D-C), ugD-gmd (D-G), ugD-fcl (D-F) and the like.

Further triple-gene enhancements galU-galE-ugD, galU-gmd-fcl and galU-manB-manC were performed, and the yield still increased with different degrees. In addition to the combinations of triple-gene enhancements listed above, many cases of triple-gene enhancement are easily conceivable, such as pgm-gulU-manA (M-U-A), pgm-galU-manB (M-U-B), pgm-galU-manC (M-U-C), pgm-galU-gmd (M-U-G), pgm-galU-fcl (M-U-F), galU-galE-manA (U-E-A) and the like.

Based on the above research, the results show that the related genes in the metabolic pathway all have a certain effect on the CA yield, so that four-gene combination enhancement (galU-galE-manB-manC (U-E-B-C), galU-galE-gmd-fcl (U-E-G-F)), five-gene combination enhancement (galU-galE-manB-manC-gmd (U-E-B-C-G), galU-galE-manB-manC-fcl (U-E-B-C-F), six-gene combination enhancement (galU-galE-manB-manC-gmd-fcl (U-E-B-C-G-F), as well as multiple- and seven-gene combination enhancement (galU-galE-ugD-manB-manC-gmd-fcl (U-E-D-B-C-G-F)) were further performed. The yield of the new engineered strain obtained by transferring the genes of different combination enhancement methods into the Δ3AB strain has increased to different degrees. Among them, the optimal engineered strain is a strain with seven genes U-E-D-B-C-G-F enhanced, named as Δ3AB-Pa.

In one embodiment, three Ptac promoters, particularly Ptac-galU-galE- ugD, Ptac-manB-manC and Ptac-gmd-fcl, are used, that is, the Ptac promoter in the first expression frame controls the expression of three genes galU-galE-ugD, the Ptac promoter in the second expression frame controls the expression of two genes manB-manC, and the Ptac promoter in the third expression frame controls the expression of two genes gmd-fcl. It should be understood by those skilled in the art that, regardless the arrangement order of different genes, the expected effect can be obtained as long as the genes are overexpressed, so the gene order is only used as an example and does not represent a limitation to the scope of the present disclosure, for example, E-D-B-C-G-F-U, D-B-C-G-F-U-E, B-C-G-F-U-E-D and the like can all be expected to have similar effects.

The selection of the genes of the precursor synthesis pathway is not limited to *E. coli per se,* and may also be from *Bacillus subtilis, Saccharomyces cerevisiae, Pichia pastoris, Kluyveromyces lactis, Streptomycetaceae* and the like; the genes with the same function are all within the scope if the sequence identity is greater than or equal to 30% determined by sequence analysis.

### 2. Component Screening and Metabolic Flow Balancing

Biological components are the cornerstones of synthetic biology, and the design and assembly of elements as well as the construction of biological element libraries are very necessary and important for the engineering of synthetic biology. With the rapid development of synthetic biology, researchers have found that various elements (promoters, ribosome binding sites, terminators, transcription factors, *etc*.) have significant effects on the efficient expression of proteins and various compounds. Promoter is a DNA sequence located upstream of the gene 5' and can be recognized by RNA polymerase, RNA is formed by transcription with DNA as a template from the transcription initiation site, and the strength of the promoter affects the transcription level. Ribosome binding site (RBS) is the initial binding site for translation, and significant up-regulation or down-regulation of translation level can be achieved by optimizing the sequence of RBS. More and more microbial gene expression elements have been analyzed in recent years, and various different element libraries have been constructed based on these elements. Based on this, various element libraries are further screened and designed further in combination with biological information technology, and the expression of the target CA is improved through combined optimization of various elements.

### 2.1 Promoter Optimization

In the work of precursor enhancement, all promoters used are the same relatively strong constitutive promoter Ptac (P1), considering that if all promoters are too strong, it would be detrimental to cell growth. Here, three different promoters are selected based on database alignment mining, and the three promoters are named as P2 to P4, respectively. The sequences corresponding to P1 to P4 are as shown in SEQ. 1 to SEQ. 4. Based on the results of the precursor synthesis module, different promoters were applied to the RcsCDB phosphate transfer and precursor pathway gene combination module to enhance the expression of the corresponding genes in the following combination manner.
P1-rcsA-rcsB-P1-galU-galE-ugD-P1-manB-manC-P1-gmd-fel (control, Δ3AB-**Pa**)
P1-rcsA-rcsB-P2-galU-galE-ugD-P3-manB-manC-P4-gmd-fcl (strain name: Δ3AB-**Pb**)
P2-rcsA-rcsB-P3-galU-galE-ugD-P4-manB-manC-P1-gmd-fcl (strain name: Δ3AB-**Pc**)
P3-rcsA-rcsB-P4-galU-galE-ugD-P1-manB-manC-P2-gmd-fcl (strain name: Δ3AB-**Pd**)
P4-rcsA-rcsB-P1-galU-galE-ugD-P2-manB-manC-P3-gmd-fcl (strain name: Δ3AB-**Pe**)

A series of engineered strains were obtained and the yields were further improved, wherein the strain Δ3AB-**Pc** had the highest yield.

It should be further noted that changing the arrangement order of the genes does not affect the yield of CA, and some different permutations and combinations are as follows.
P1-gmd-fcl-P2-rcsA-rcsB-P3-galU-galE-ugD-P4-manB-manC (strain name: Δ3AB-**Pf**)
P4-manB-manC-P1-gmd-fcl-P2-rcsA-rcsB-P3-galU-galE-ugD (strain name: Δ3AB-**Pg**)
P3-galU-galE-ugD-P4-manB-manC-P1-gmd-fcl-P2-rcsA-rcsB (strain name: Δ3AB-**Ph**)
P2-rcsA-rcsB-P3-galU-galE-ugD-P4-manB-manC-P1-gmd-fcl (strain name: Δ3AB-**Pc**)

In addition, there are many different permutations and combinations to achieve similar effects. The above results also indicate that the expression frames P2-rcsA-rcsB, P3-galU-galE-ugD, P4-manB-manC and P1-gmd-fcl can achieve the effect as long as they are expressed.

### 2.2 Screening and Optimization of RBS Sequences

The RBS sequences corresponding to the above promoter optimization are all identical (aagaagaga), and considering that the difference of the RBS sequences and the length of the sequences significantly affect the expression of genes, an RBS degenerate sequence NNNNNNNNNN with a length of 10 bp is designed here. The effect of differences in RBS sequences on green fluorescent protein expression was verified based on the P1 promoter. After sorting according to RBS strength, the first 90 sequences were selected for subsequent studies. The 9 highest-strength sequences, ranked 1-9, were selected and named as R_{H1}~R_{H9}, the 9 medium-strength sequences, ranked 41-49, were selected and named as R_{M1}~R_{M9}, and the 9 low-strength sequences, ranked 82-90, were selected and named as R_{M1}~R_{L9}.

### 2.3 Optimization of Promoters and RBS Combination to Improve Strain Performance

RBS in the above strain Δ3AB-Pc-A (P2-rcsA-rcsB-P3-galU-galE-ugD-P4-manB-manC-P1-gmd-fcl) was replaced with all the highest-strength RBS sequences R_{H1}~R_{H9}, or medium-strength sequences R_{M1}~R_{M9}, or low-strength sequences R_{L1}~R_{L9}, or combinations of different strength, respectively. It should be noted that in prokaryotes, although multiple genes can share the same promoter, one RBS is required before each gene. The combinations of RBSs with different strength are as follows.
R_{H1}-R_{H2}-R_{H3}-R_{H4}-R_{HS}-R_{H6}-R_{H7}-R_{HS}-R_{H9} (Δ3AB-Pc-**Ra**)
R_{M1}-R_{M2}-R_{M3}-R_{M4}-R_{M5}-R_{M6}-R_{M7}-R_{M8}-R_{M9} (Δ3AB-Pc-**Rb**)
R_{L1}-R_{L2}-R_{L3}-R_{L4}-R_{L5}-R_{L6}-R_{L7}-R_{L8}-R_{L9} (Δ3AB-Pc-**Rc**)
R_{H1}-R_{H2}-R_{H3}-R_{H4}-R_{H5}-R_{H6}-R_{H7}-R_{M8}-R_{M9} (Δ3AB-Pc-**Rd**)
R_{H1}-R_{H2}-R_{M3}-R_{M4}-R_{M5}-R_{M6}-R_{M7}-R_{M8}-R_{M9} (Δ3AB-Pc-**Re**)
R_{M1}-R_{M2}-R_{M3}-R_{M4}-R_{M5}-R_{H6}-R_{H7}-R_{H8}-R_{H9} (Δ3AB-Pc-**Rf**)
R_{M1}-R_{M2}-R_{H3}-R_{H4}-R_{H5}-R_{H6}-R_{H7}-R_{H8}-R_{H9} (Δ3AB-Pc-**Rg**)
R_{H1}-R_{H2}-R_{L3}-R_{L4}-R_{L5}-R_{H6}-R_{H7}-R_{L8}-R_{L9} (Δ3AB-Pc-**Rh**)
R_{H1}-R_{H2}-R_{M3}-R_{M4}-R_{M5}-R_{M6}-R_{M7}-R_{L8}-R_{L9} (Δ3AB-Pc-**Ri**)
R_{H1}-R_{H2}-R_{L3}-R_{L4}-R_{L5}-R_{M6}-R_{M7}-R_{L8}-R_{L9} (Δ3AB-Pc-**Rj**)

The names of the corresponding strains are shown in parentheses. The series of strains were subjected to a fermentation test, wherein the strain Δ3AB-Pc-Rj-Ac had the highest fermentation yield.

The listed combinations are only a portion of possible combinations, and many different combinations may be formed based on the strategy to achieve a similar effect.

### 3. Gene Integration Strategy to Improve CA Yield

a) knocking out lipopolysaccharide core polysaccharide synthesis gene cluster waaL, waaU, waaZ, waaY, waaR, waaO, waaB, waaP, waaG, and waaQ in the genome, as well as Lon protein coding gene lon and HNS regulatory protein coding gene hns;
b) knocking out acetic acid synthesis pathway gene poxB;
c) integrating and expressing DNA binding transcription activator coding gene rcsA and DNA binding transcription activator coding gene rcsB at the genome level;
d) further integrating and expressing isocitrate dehydrogenase gene icd related to NADPH regeneration and/or pyridine nucleotide transhydrogenase coding gene pntAB; integrating and expressing guanosine kinase coding gene gsk related to GTP regeneration;
e) further integrating and expressing UTP-1-glucose phosphate uridylyltransferase coding gene galU;
f) further integrating and expressing UDP-glucose-1-phosphotransferase coding gene wcaJ and/or colanic acid polymerase coding gene wcaD related to colanic acid synthesis cluster.

### Materials and Methods Used in Specific Examples

Seed medium: yeast powder 5-10 g/L, tryptone 5-10 g/L, sodium chloride 5-10 g/L.

Fermentation medium: yeast powder 10-20 g/L, tryptone 20-40 g/L, KH₂PO₄ 2-4 g/L, K₂HPO₄ 10-15 g/L, glucose 10-30 g/L, pH 4.5-7.5.

Molecular cloning related reagents: seamless cloning enzyme (Takara), DNA polymerase (Takara), DNA recovery kit (Shanghai Biotech), plasmid extraction kit (Shanghai Biotech), DNA marker (Takara).

Seed culture method: single colonies streaked on a flat plate were picked with an inoculation loop and inoculated into 3-7 mL seed medium, and cultured at 34-38 °C for 10-18 hours.

Culture method of shake flask fermentation test: the seed broth was inoculated into the fermentation medium at an inoculation amount of 1% - 10%, and the inoculated fermentation medium was cultured at 25-32 °C for 48 hours.

Polysaccharide detection method: (1) Sample treatment. After 48 hours of culture, the fermentation broth was centrifuged at a high speed of 10,000-15,000 rpm for 20 minutes in a refrigerated centrifuge, and the supernatant was collected; 1.5-4 times anhydrous ethanol was added to the supernatant, and the mixture was allowed to stand overnight in a refrigerator at 2-8°C. The alcohol-precipitated solution was centrifuged at high speed for 20 minutes, the supernatant was removed, and the precipitate was retained; an appropriate amount of sterile water was added to redissolve the precipitate, and the obtained solution was the extracted polysaccharide extract. (2) CA Yield Test. First, preparing different concentrations of fucose standards: 0, 1, 2, 3, 4, 5, 6, 7, 8, 9, 10 g/L; taking 50 µL of different concentrations of fucose standards, adding 4.5 mL of sulfuric acid solution (H₂SO₄/H₂O (6:1; v/v).), subjected to boiling water bath for 20 minutes, naturally cooling to room temperature, measuring OD₃₉₆ and OD₄₂₇ respectively with a microplate reader, then adding 100 µL of 1 mol/L Cysteine hydrochloride solution, measuring OD'396 and OD'427 again, and calculating ΔOD396 (OD'396 - OD396) and ΔOD427 (OD'427 - OD427). Using the fucose concentration as an abscissa and ΔOD396- ΔOD427 as an ordinate, a standard curve of the fucose concentration was drawn, and a concentration conversion equation was calculated. When measuring the sample concentration, 50 µL of a diluted sample with an appropriate concentration was detected by the same procedure, and the concentration of CA was calculated by calibrating the concentration of fucose in the sample using the fucose standard curve corresponding to the ΔOD396 - ΔOD427 value.

### EXAMPLES

The following examples are merely illustrative and are not intended to limit the scope or content of the invention in any way.

### Example 1. Construction of Lipopolysaccharide Pathway Single-Gene Knockout Strain

The synthesis of lipopolysaccharide consumes the precursor substance, knocking out the gene (or gene cluster) in this pathway is considered, in a specific example, an exemplary starting strain is *Escherichia coli* str. K-12 substr. MG1655, with gene waaF (gene ID: 948135), gene cluster LG (comprising genes waaL (gene ID: 948148), waaU (gene ID: 948147), waaZ (gene ID: 948146), waaY (gene ID: 948145), waaR (gene ID: 948142), waaO (gene ID: 948143), waaB (gene ID: 948144), waaS (gene ID: 948151), waaP (gene ID: 948150), waaG (gene ID: 948149)) or gene cluster LQ (waaL (gene ID: 948148), waaU (gene ID: 948147), waaZ (gene ID: 948146), waaY (gene ID: 948145), waaR (gene ID: 948142), waaO (gene ID: 948143), waaB (gene ID: 948144), waaS (gene ID: 948151), waaP (gene ID: 948150), waaG (gene ID: 948149), waaQ (gene ID: 948155)) knocked out, respectively. The genome genes were knocked out by CRISPR-Cas9 method.

### 1. Construction of Homologous Arm Fragment and sgRNA Plasmid

In order to perform knockout of relevant genes, the corresponding 1000 bp nucleotide sequences on the upstream and downstream of the gene (or gene cluster) were first amplified by PCR, respectively named as waaF-arm1, waaF-arm2; LG-arm1, LG-arm2; LQ-arm1, LQ-arm2. The primers used were named as waaF-arm1-F/R, waaF-arm2-F/R; LG-arm1-F/R, LG-arm2-F/R; LG-arm1-F/LQ-arm1-R, LQ-arm2-F/R, respectively, and the specific sequences are shown in Table 2.

**Table 2. The primers and sgRNA sequences required for the construction of engineered strains**

| Primer Name | Sequences (5'-3') | SEQ ID NO |
|---|---|---|
| waaF-arm1-F | atgaatatgttcgtcaaatcctgcctgaagc | 32 |
| waaF-arm1-R | gttttaacgatcaaaacccgcatccggcagagctcttatgcgtcgcgattc | 33 |
| waaF-arm2-F | gaatcgcgacgcataagagctctgccggatgcgggttttgatcgttaaaacatc | 34 |
| waaF-arm2-R | gcgatagcataatcgccctgggtttg | 35 |
| LG-arml-F | cgcactattgttacaagaggaagcctg | 36 |
| LG-arm1-R | cttcapctgacaggaatgcacaattgttttacctttataatgatgataacttttccaaaactgcttgactg | 37 |
| LG-arm2-F | gttatcatcattataaaggtaaaacaattgtgcattcctgtcagctgaaggg | 38 |
| LG-arm2-R | ctcctgtcatcagtacgctcaagcagaattatc | 39 |
| LQ-arm1-R | atttcgaaaaatcctggtcataaaggttttacctttataatgatgataacttttccaaaactgcttgactg | 40 |
| LQ-arm2-F | gttatcatcattataaaggtaaaacctttatgaccaggatttttcgaaatggcttttccac | 41 |
| LQ-arm2-R | cttcttcattttgcgcagcaatcagcg | 42 |
| hns-arml-F | ctatcattacaactgcctcgcttgttataagcg | 43 |
| hns-arml-R | accttgctcatccattccttcttccagcgtttcaagtgtacattctcttgc | 44 |
| hns-arm2-F | tacacttgaaacgctggaagaagcaatggatgagcaaggtaaatccctc | 45 |
| hns-arm2-R | cgctattatgggtcgttacgtacttagc | 46 |
| rcsA-F | catcaccacagccaggatccgatgtcaacgattattatggatttatgtagttacacccg | 47 |
| rcsA-R | cattatgcggccgcaagcttttagcgcatgttgacaaaaataccattagtcac | 48 |
| rcsB-F | aaggagatataccatataccatgaacaatatgaacgtaattattgccgatgacca | 49 |
| rcsB-R | ccgagctcgaattcggatccttagtctttatctgccggacttaagatcact | 50 |
| pgi-F | aaggagatataccatataccatgaaaaacatcaatccaacgcagaccgc | 51 |
| pgi-R | cgccgagctcgaattcggatccttaaccgcgccacgctttatagcggttaatca | 52 |
| pgm-F | aaggagatataccatataccatggcaatccacaatcgtgcaggc | 53 |
| pgm-R | cgccgagctcgaattcggatccttacgcgtttttcagaacttcgctaacaa | 54 |
| galU-F | aaggagatataccatataccatggctgccattaacgaaagtcaaaaaag | 55 |
| galU-R | cgccgagctcgaattcggatccttacttcttaatgcccatctcttcttcaagc | 56 |
| alE-F | aaggagatataccatataccatgagagttctggttaccggtggtag | 57 |
| galE-R | cgccgagctcgaattcggatccttaatcgggatatccctgtggatggcgt | 58 |
| ugD-F | aaggagatataccatataccatgaaaatcaccatttccggtactggc | 59 |
| ugD-R | cgccgagctcgaattcggatccttagtcgctgccaaagagatcgcggg | 60 |
| manA-F | aaggagatataccatataccatgcaaaaactcattaactcagtgcaaaac | 61 |
| manA-R | cgccgagctcgaattcggatccttacagcttgttgtaaacacgcgctaaa | 62 |
| manB-F | aaggagatataccatataccatgaaaaaattaacctgctttaaagcctatg | 63 |
| manB-R | cgccgagctcgaattcggatccttactcgttcagcaacgtcagcag | 64 |
| manC-F | agaaggagatataccatataccatggcgcagtcgaaactctatcc | 65 |
| manC-R | cgccgagctcgaattcggatccttacacccgtccgtagcgatccgcg | 66 |
| gmd-F | aaggagatataccatataccatgtcaaaagtcgctctcatcaccg | 67 |
| gmd-R | cgccgagctcgaattcggatccttatgactccagcgcgatcgcc | 68 |
| fcl-F | aaggagatataccatataccatgagtaaacaacgagtttttattgctg | 69 |
| fcl-R | cgccgagctcgaattcggatccttacccccgaaagcggtcttg | 70 |
| sgRNA Plasmids | sgRNA Sequences (5'-3') | - |
| pTarget-waaF | ctgctgctcggtattcaaag | 71 |
| pTarget-LG | actagtattatacctaggactgagc | 72 |
| pTarget-LQ | tgtgtagatgagattgcacg | 73 |
| pTarget-hns | acgtcgcgaagaagaaagcg | 74 |
| pTarget-lon | ggatcaggcaagaaaccagcg | 75 |

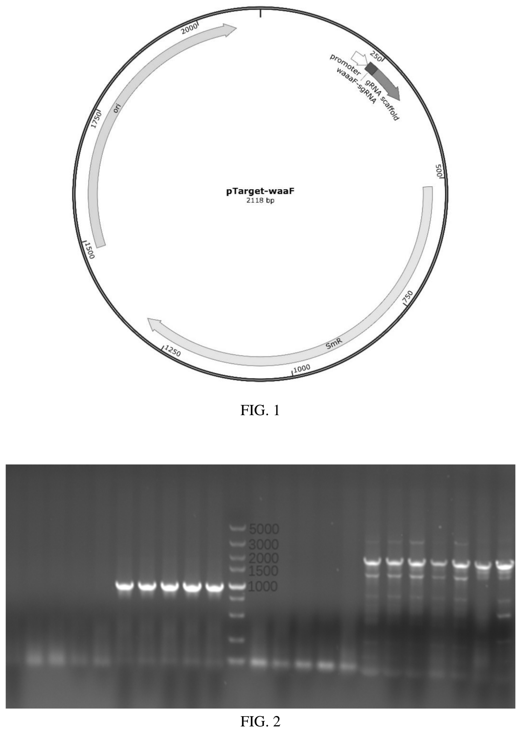

Two corresponding upstream and downstream homologous arms were connected into a fragment waaF, LG-arm, LQ-arm by fusion PCR. Then, in order to construct the sgRNA, the sgRNA sequence was designed online by using the chopchop website with the corresponding gene sequence as a template, and the obtained sequence was ligated to the pTarget plasmid to obtain plasmids containing the sgRNAs of the corresponding genes, which were pTarget-waaF, pTarget-LG and pTarget-LQ, respectively. The corresponding pTarget-waaF plasmid map is shown in FIG. 1, and the other two maps are similar.

### 2. Preparation of E. coli MG1655 Competent Cells

In order to perform gene knockout, the constructed homologous arm fragments and sgRNA plasmids need to be introduced into cells, and thus the cells need to be made competent to absorb exogenous DNA. (1) The strain containing pCas9 plasmid was inoculated into 50 mL of LB medium at an inoculation amount of 1%~2%, and cultured at 30 °C; when OD₆₀₀ grew to 0.1~0.2, 2 mL of L-arabinose (final concentration 40 mM) with a concentration of 1 M was added, and the strain were cultured at 30 °C to induce expression of recombinant enzyme. The induction time was at least 1 hour, and then preparation of competent cells began once OD₆₀₀ reached 0.6~0.7. (2) The cells were collected by centrifugation at 5000 rpm, 4 °C for 5 minutes, and then resuspended with precooled sterile 10% glycerol and washed 3 times (washing was performed with 1 mL glycerol solution). (3) Finally, 500 µL of pre-cooled 10% glycerol was added (50 mL of bacterial broth was concentrated 100-fold) to prepare competent cells, and the cells were aliquoted into 90 µL per tube and frozen at -80 °C for later use.

### 3. Electrotransformation Knockout

The obtained homologous arm fragments waaF-arm, LG-arm, LQ-arm and sgRNA pTarget-waaF, pTarget-LG, pTarget-LQ plasmids were added to competent cells consecutively at a ratio of 1:1~1:4, mixed gently, and subjected to ice bath for 30 minutes; the cells were transferred to an electrotransformation cup for electrotransformation (1 mm electrotransformation cup, 1800V, 200 Ω), 600 µL of pre-cooled LB medium was added immediately after electrotransformation, and the cells were transferred to a 1.5 mL EP tube, and cultured at 30°C for 2.5~3 hours. The cells were then plated on spectinomycin- and kanamycin-resistant plates and cultured at 30 °C until colony formation, and then PCR verification of the knockout results was performed, as shown in Figure 2, which showed the results of the verification before and after the knockout of waaF and LQ, and the absence of bands indicated that the knockout was successful.

### 4. Elimination of Plasmids

The selected successfully eliminated strains were inoculated to 2 mL of LB solution (with the corresponding antibiotic Kan added), IPTG was added to a final concentration of 0.5 mM, and the strains were cultured at 30 °C for 12 hours to eliminate the pTarget plasmid. A small amount of the bacterial broth was streaked on a Kan plate and cultured at 30 °C, and the grown single colonies were picked up and spotted separately to Spc plate and Kan plate, respectively. Strains that could grow on Kan plates but not on Spc plates were those that had eliminated the pTarget plasmid. Single colonies were picked and inoculated into an LB medium, and cultured at 37 °C (also possible at 42 °C) overnight (the temperature-sensitive plasmid pCas will be lost at a high temperature), a small amount of bacterial broth was taken and streaked on an antibiotic-free plate, and cultured at 37 °C. Single colonies were spotted on a Kan plate and an antibiotic-free plate, cultured at 37 °C, and strains that could grow on antibiotic-free plates but not on Kan plates were those that had eliminated the pTarget plasmid. Finally, strains ΔF, ΔLG, ΔLQ with relevant genes knocked out were obtained.

### Example 2 Verification of Lipopolysaccharide Pathway Gene Knocked-out Strain

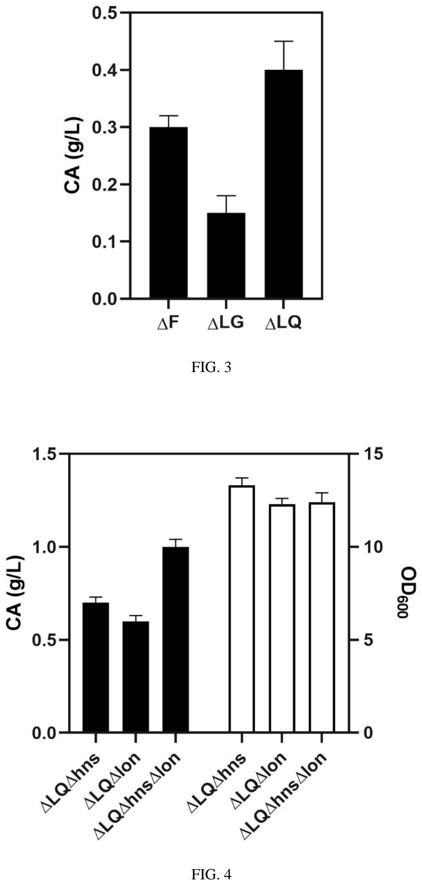

The strains ΔF, ΔLG, ΔLQ obtained by the above knockout were inoculated in a seed medium for culture, and then transferred to a fermentation medium for fermentation testing. The obtained fermentation broth was centrifuged at a high speed to collect the supernatant of the fermentation broth, and the yields of the corresponding three strains were detected by the chromogenic method, which were 0.3 g/L, 0.15 g/L, 0.4 g/L, respectively (FIG. 3). The results show that knockout of genes related to lipopolysaccharide pathway has a significant promoting effect on the synthesis of CA, wherein knockout of gene cluster LQ has the highest yield.

### Example 3 Knockout of Related Genes in ResCDB Module

Using the same technical means as in Example 1, homologous arms hns-arm, lon-arm with hns (gene ID: 945829) and Ion (gene ID: 945085) knocked-out, respectively, as well as sgRNA plasmids pTarget-hns, pTarget-lon were constructed based on the ΔLQ strain; related primer design sequences are shown in Table 2. Competent preparation-electrotransformation knockout-plasmid elimination steps were performed in the same way to obtain strains ΔLQΔhns and ΔLQΔlon. Then, the strains were analyzed and verified by shake flask, the corresponding yields were shown in FIG. 4, and the knockout of two genes significantly improved the yields of CA, which were 0.7 g/L and 0.6 g/L, respectively. Therefore, the lon was further knocked-out based on the ΔLQΔhns strain, and the yield of the iteratively knocked-out strain ΔLQΔhnsΔlon (Δ3) reached 1.0 g/L, as shown in FIG. 4. The technical strategy shows that the improvement of CA yield can be effectively achieved by knocking out genes related to the RcsCDB module. At the same time, the growth states of the three engineered strain were analyzed, and as shown in FIG. 4, the growth states of the three strains were not greatly different from each other and were not significantly affected.

### Example 4 Overexpression of Related Genes in the RcsCDB Module

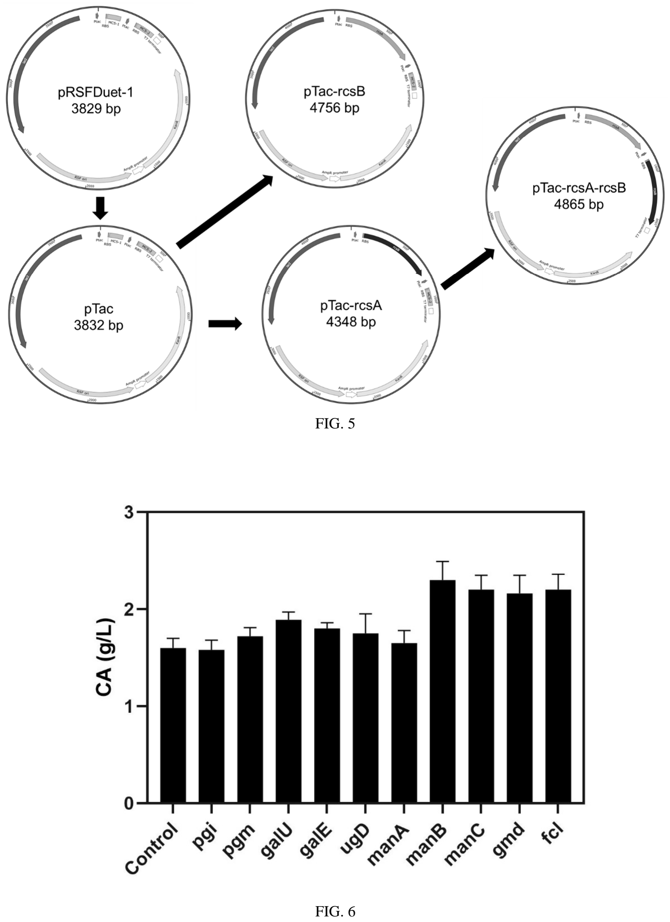

Through analysis, it is found that rcsA and rcsB play an important role in regulating the synthesis of CA, so rcsA and rcsB are separately overexpressed or rcsA-rcsB are overexpressed in combination, and changes in the yields of different engineered strains are investigated. Using *E. coli* genome as a template, rcsA (gene ID: 946467) and rcsB (gene ID: 947441) were amplified by PCR, and the corresponding primers were rcsA-F/R and rcsB-F/R, respectively. Using the pRSFDuet-1 plasmid as the backbone, the two T7 promoters on the plasmid were replaced with two Ptac promoters by PCR, and the newly constructed plasmid was named pTac. Then, the amplified rcsA and rcsB genes were ligated to plasmids respectively to obtain pTac-rcsA and pTac-rcsB; and then the rcsB gene fragment was ligated to the plasmid with pTac-rcsA plasmid as a template to obtain pTac-rcsA-rcsB plasmid. The construction process is shown in FIG. 5.

The strain Δ3 obtained from the previous construction was inoculated into a seed medium, and the strain was prepared into competent cells capable of chemical transformation by a universal competent preparation method. Then, plasmids pTac-rcsA, pTac-rcsB and pTac-rcsA-rcsB were respectively transformed, and the corresponding yields of the obtained strains were verified by shaking flask analysis, which were 1.4 g/L, 0.9 g/L and 1.6 g/L, respectively. The results show that when rcsA was overexpressed alone or rcsA-rcsB were expressed in combination, the yield was significantly increased, while when rcsB was overexpressed alone, the yield was not increased, but slightly decreased. Studies show that rcsA regulates the gene cluster of CA synthesis, so the overexpression of this gene increases the yield, which is consistent with the research, while rcsB is closely related to CA synthesis, but the overexpression of rcsB alone decreases the yield, and, when rcsB and rcsA are overexpressed simultaneously, the yield increases. It is speculated that rcsB needs to work in conjunction with rcsA to function properly, and at the same time, rcsB may also be related to the regulation of other genes in cells, and overexpression of rcsB alone may cause intracellular metabolic abnormalities, which in turn leads to a decrease in the synthesis of the target product CA. The above strain overexpressing rcsA-rcsB was named as Δ3AB.

### Example 5 Single-Gene Enhanced Expression of Precursor Synthesis Pathway

Using *E. coli* genome as a template, the precursor synthesis pathway genes pgi (gene ID: 948535), pgm (gene ID: 945271), galU (gene ID: 945730), galE (gene ID: 945354), ugD (gene ID: 946571), manA (gene ID: 944840), manB (gene ID: 946574), manC (gene ID: 946580), gmd (gene ID: 946562), fcl (gene ID: 946804) were amplified; the corresponding primers are shown in Table 2. Using the pTac plasmid constructed in Example 4 as a skeleton, these genes were respectively ligated to the plasmid to obtain recombinant plasmids pTac-pgi, pTac-pgm, pTac-galU, pTac-galE, pTac-ugD, pTac-manA, pTac-manB, pTac-manC, pTac-gmd, pTac-fcl; the series of plasmids were respectively transferred into the Δ3AB strain, and the obtained recombinant strains were analyzed and verified by shake flask, and the corresponding yields are shown in FIG. 6. The results show that there are different degrees of increase in CA yield when the expression of pgm, galU, galE, ugD, manA, manB, manC, gmd or fcl is enhanced alone, whereas there is no increase in CA yield when pgi is enhanced.

### Example 6 Dual-Gene Enhanced Expression of Precursor Synthesis Pathway

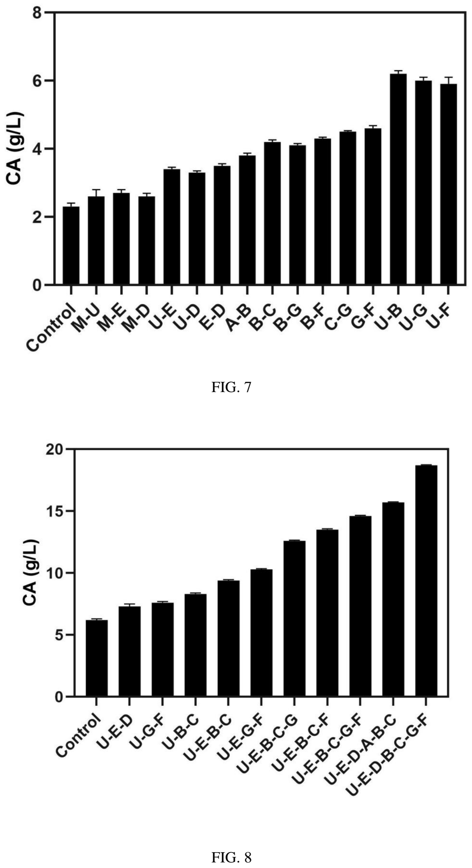

Based on the results of Example 5, genes that can promote CA synthesis were further combined in pairs, and the following combinations were selected: pgm-galU (M-U), pgm-galE (M-E), pgm-ugD (M-D), galU-galE (U-E), galU-ugD (U-D), galE-ugD (E-D), manA-manB (A-B), manB-manC (B-C), manB-gmd (B-G), manB-fcl (B-F), manC-gmd (C-G), gmd-fcl (G-F), galU-manB (U-B), galU-manC (U-C), galU-gmd (U-G), galU-fcl (U-F), which were constructed on the pTac plasmid, the obtained recombinant plasmids were transferred into Δ3AB strain, and the obtained recombinant strains were subjected to shake flask verification analysis, with the corresponding yield shown in FIG. 7. The results show that the enhanced expression of galU-manB leads to the highest yield, reaching 6.2 g/L; the overall results show that when two genes are enhanced in series, if a single-side precursor pathway is enhanced, there is a certain limit on the improvement of the yield, and when two pathways are enhanced at the same time, such as U-B, U-C, U-G and U-F, the overall expression amount is higher than that of single-side enhanced genes in series. The results show that not only gene overexpression but also the balance of metabolic flow in different pathways should be considered.

### Example 7 Multi-Gene Combination Enhanced Expression of Precursor Synthesis Pathway

The effects on the CA yield were further examined by means of triple-gene enhancements galU-galE-ug D(U-E-D), galU-gmd-fcl (U-G-F), galU-manB-manC (U-B-C); four-gene combination enhancements galU-galE-manB-manC (U-E-B-C), galU-galE-gmd-fcl (U-E-G-F); five-gene combination enhancements galU-galE-manB-manC-gmd (U-E-B-C-G), galU-galE-manB-manC-fcl (U-E-B-C-F); six-gene combination enhancements galU-galE-manB-manC-gmd-fcl (U-E-B-C-G-F), galU-galE-ugD-manA-manB-manC (U-E-D-A-B-C); and seven-gene combination enhancements galU-galE-ugD-manB-manC-manC-gmd-fcl (U-E-D-B-C-G-F), galU-galE-ugD-manA-manB-manC-gmd (U-E-D-A-B-C-G). The results are shown in FIG. 8, wherein the control group is a dual-gene enhanced galU-manB strain, and the results show that the highest yield reached 8.3 g/L when three genes were overexpressed (U-B-C), the yield was further increased to 10.3 g/L when four genes were overexpressed (U-E-G-F), the highest yield was 13.5 g/L when five genes were overexpressed (U-E-B-C-F), and finally the highest yield reached 18.7 g/L when seven genes were overexpressed (U-E-D-B-C-G-F), and the optimal strain was named as Δ3AB-Pa. The results show that different gene combinations can increases CA yield in different degrees.

The exemplified combinations are only a portion of all combinations, and other different combinations can also achieve similar effects.

### Example 8 Effect of Gene Arrangement Order on CA Yield

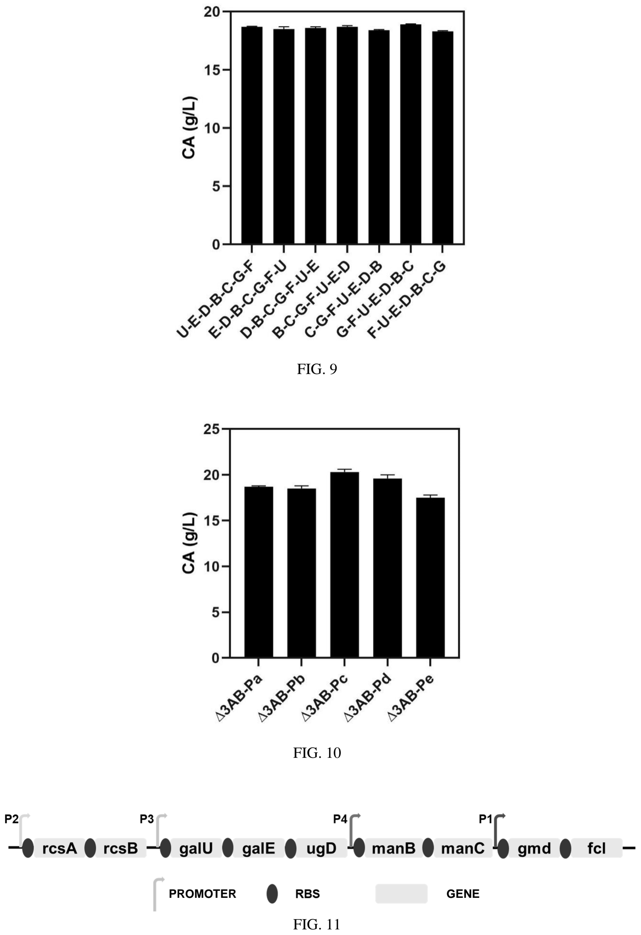

Based on the optimal seven-gene combination galU-galE-ugD-manB-manC-gmd-fcl (U-E-D-B-C-G-F) obtained above, the CA has the highest yield. These genes were arranged in different orders E-D-B-C-G-F-U, D-B-C-G-F-U-E, B-C-G-F-U-E-D, C-G-F-U-E-D-B, G-F-U-E-D-B-C and F-U-E-D-B-C-G, and the obtained engineered strains were fermented and then subjected to CA yield detection, and the results are shown in FIG. 9. In these different arrangement order combinations, there are no significant differences in the yields of CA, so the results show that similar effects can be obtained by expressing these genes in different order.

### Example 9 Optimization of Promoter to Improve CA Synthesis

In the previous work, the yield is improved by overexpression of the precursor pathway, but the the pTac promoter (P1) is used in the overexpression of all genes, and since that different promoters have a significant effect on the expression of genes, three promoters in *E. coli* are selected here, named as P2 to P4, respectively, wherein the sequences corresponding to P1 to P4 are shown in SEQ ID NOs: 1-4. The expression frame of the optimal strain Δ3AB-Pa was constructed as follows: Pl-rcsA-rcsB-P1-galU-galE-ugD-Pl-manB-manC-Pl-gmd-fcl, and pTac promoter (P1) was used in each expression frame of this strain, and three different promoters were applied to this strategy to construct the following combined enhanced strains: P1-rcsA-rcsB-P2-galU-galE-ugD-P3-manB-manC-P4-gmd-fcl (strain name: Δ3AB-Pb), P2-rcsA-rcsB-P3-galU-galE-ugD-P4-manB-manC-P1-gmd-fcl (strain name: Δ3AB-Pc), P3-rcsA-rcsB-P4-galU-galE-ugD-Pl-manB-manC-P2-gmd-fcl (strain name: Δ3AB-Pd), and P4-rcsA-rcsB-P1-galU-galE-ugD-P2-manB-manC-P3-gmd-fcl (strain name: Δ3AB-Pe). A series of engineered strains with varying degrees of increase in yield were obtained, wherein the yield of strain Δ3AB-Pc was the highest, reaching 16 g/L, and the results are shown in FIG. 10.

The promoters are not limited to P1 to P4, and it can be appreciated that promoter sequences from other different sources can be used, and similar effects can be achieved through certain optimization.
SEQ ID NO:1: ttgacaattaatcatcggctcgtataatgtgatcagacctttgtttaactttaagaaggagatatacc
SEQ ID NO:2: ttgacagctagctcagtcctaggtataatactagt
SEQ ID NO:3: taatacgactcactatagg
SEQ ID NO:4: gtttatacataggcgagtactctgttatgg

### Example 10. Screening of RBS Sequences

The expression frame corresponding to the enhanced expression of the engineered strain Δ3AB-Pc obtained in Example 9 is described in more detail in FIG. 11. As can be seen from the figure, the expression frame contains 4 different promoters, wherein each gene has an identical RBS with the sequence of aagaagaga preceding the gene. Since RBS is a ribosome binding site, the difference in this sequence will significantly affect its binding to ribosomes and affect the translation process of ribosomes, resulting in a difference in gene expression. Therefore, here we preformed screening of the RBS sequences.

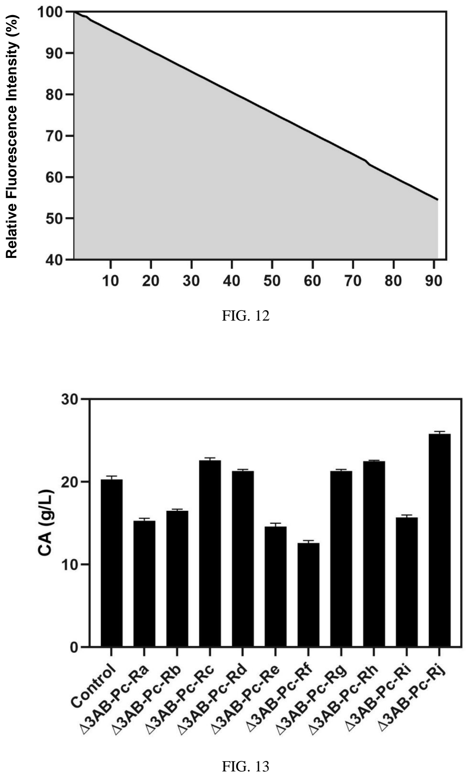

First, the green fluorescent protein gene egfp was inserted into the pTac plasmid to obtain the pTac-eGFP plasmid, and then degenerate primers were designed using the pTac-eGFP plasmid as a template; after amplification with the degenerate primers 10RBS-F/R, a plasmid library containing different RBS sequences was obtained. The plasmid library was transferred into *Escherichia coli,* and several colonies grown were picked into 96-well plates by an automatic colony picker, and a total of about 40,000 single colonies were picked. Then, plates were cultured under the same conditions, and the corresponding relative fluorescence intensity in each plate was recorded after 24 h of culture. 90 strains with high to low fluorescence intensity were selected, and the relative fluorescence intensity was shown in FIG. 12. Nine RBS sequences with the highest fluorescence intensity, ranked of 1-9, were selected and named as R_{H1}~R_{H9}, 9 medium-strength RBS sequences, ranked 41-49 were selected and named as R_{M1}~R_{M9}, and 9 low-strength RBS sequences, ranked of 82-90, were selected and named as R_{L1}~R_{L9}, and the RBS sequences of the 27 strains were sequenced, and the obtained sequence results are shown in Table 1.

**Table 1. Different RBS Sequences**

| Name | Sequence | SEQ ID NO | Name | Sequence | SEQ ID NO | Name | Sequence | SEQ ID NO |
|---|---|---|---|---|---|---|---|---|
| R_{H1} | aagaagggga | 5 | R_{M1} | aggtaggggc | 14 | R_{L1} | aaagattaga | 23 |
| R_{H2} | aagaagagga | 6 | R_{M2} | aggtaggtga | 15 | R_{L2} | aaagatatca | 24 |
| R_{H3} | aagaaggggc | 7 | R_{M3} | aggtagtgga | 16 | R_{L3} | aagagtagta | 25 |
| R_{H4} | aagaggggga | 8 | R_{M4} | aggtagcgga | 17 | R_{L4} | aaaagggata | 26 |
| R_{H5} | ggaaagggga | 9 | R_{M5} | aggcgaggga | 18 | R_{L5} | agagagaata | 27 |
| R_{H6} | agagagggga | 10 | R_{M6} | aggacaggga | 19 | R_{L6} | gagaggcaga | 28 |
| R_{H7} | aagaagggag | 11 | R_{M7} | aggtaggggt | 20 | R_{L7} | gagacaagga | 29 |
| R_{H8} | aggtagggga | 12 | R_{M8} | cggtagggga | 21 | R_{L8} | gggaaagaga | 30 |
| R_{H9} | aagagaggag | 13 | R_{M9} | atagagggga | 22 | R_{L9} | gaggacggag | 31 |

### Example 11. Optimization of Promoter and RBS Combinations for Balancing Metabolic Flow

RBS usage corresponding to strain Δ3AB-Pc (P2-rcsA-rcsB-P3-galU-galE-ugD-P4-manB-manC-P1-gmd-fcl) in Example 9 is shown in FIG. 11. Since the same RBS is used preceeding all genes, the strains obtained by this construction method contains multiple identical sequences, and the stability of the strains are poor; on the other hand, the expression of all genes is either too strong or too weak due to the identical RBS, which is not conducive to metabolic flow balance and product synthesis. Here, the RBS in the Δ3AB-Pc strain was replaced with all the highest-strength RBS sequences R_{H1}~R_{H9}, or the medium-strength sequences R_{M1}~R_{M9}, or the low-strength sequences R_{L1}~R_{L9}, or a combination of different strengths. The combinations of different RBSs are as follows.
R_{H1}-R_{H2}-R_{H3}-R_{H4}-R_{HS}-R_{H6}-R_{H7}-R_{H8}-R_{H9} (Δ3AB-Pc-**Ra**)
R_{M1}-R_{M2}-R_{M3}-R_{M4}-R_{M5}-R_{M6}-R_{M7}-R_{M8}-R_{M9} (Δ3AB-Pc-**Rb**)
R_{L1}-R_{L2}-R_{L3}-R_{L4}-R_{L5}-R_{L6}-R_{L7}-R_{L8}-R_{L9} (Δ3AB-Pc-**Rc**)
R_{H1}-R_{H2}-R_{H3}-R_{H4}-R_{H5}-R_{H6}-R_{H7}-R_{M8}-R_{M9} (Δ3AB-Pc-**Rd**)
R_{H1}-R_{H2}-R_{M3}-R_{M4}-R_{M5}-R_{M6}-R_{M7}-R_{M8}-R_{M9} (Δ3AB-Pc-**Re**)
R_{M1}-R_{M2}-R_{M3}-R_{M4}-R_{M5}-R_{H6}-R_{H7}-R_{H8}-R_{H9} (Δ3AB-Pc-**Rf**)
R_{M1}-R_{M2}-R_{H3}-R_{H4}-R_{H5}-R_{H6}-R_{H7}-R_{H8}-R_{H9} (Δ3AB-Pc-**Rg**)
R_{H1}-R_{H2}-R_{L3}-R_{L4}-R_{L5}-R_{H6}-R_{H7}-R_{L8}-R_{L9} (Δ3AB-Pc-**Rh**)
R_{H1}-R_{H2}-R_{M3}-R_{M4}-R_{M5}-R_{M6}-R_{M7}-R_{L8}-R_{L9} (Δ3AB-Pc-**Ri**)
R_{H1}-R_{H2}-R_{L3}-R_{L4}-R_{L5}-R_{M6}-R_{M7}-R_{L8}-R_{L9} (Δ3AB-Pc-**Rj**)

The names of the corresponding strains are shown in parentheses. The series of strains were subjected to a fermentation test, wherein the strain Δ3AB-Pc-Rj had the highest fermentation yield, reaching 25.8 g/L, as shown in FIG. 13. In this optimal strain, the rcsA-rcsB genes were expressed with the highest-strength RBS sequence (RH1-RH2), while the three genes galU-galE-ugD were expressed with three low-strength RBSs (RL3-RL4-RL5), and the following galU-galE-ugD were expressed with medium-strength RBSs (RM6-RM7), and gmd-fcl was expressed with low-strength RBS (RL8-RL9). For strain Δ3AB-Pc-Ra, although the used RBS sequences were all highest strength, the yield was only 15.3 g/L, which was lower as compared to the control. The results show that the strength and combination of RBS sequences have a significant impact on CA yield, and a significant improvement in CA yield can be achieved by screening and optimizing RBS sequences.

The listed combinations are only a portion of possible combinations, and many different combinations may be formed based on the strategy, to achieve a similar effect.

In the case where the starting strains are *E. coli* BL21 (DE3), JM109, Nissle 1917 (EcN), BW23110 or MG1655, respectively, the gene knockout or gene overexpression, promoter optimization and ribosome binding site optimization of Examples 1-12 were repeated, and the obtained optimized strains can achieve significant improvement of CA yield.

### Example 12. Knockout of Acetic Acid Synthesis Pathway Gene poxB

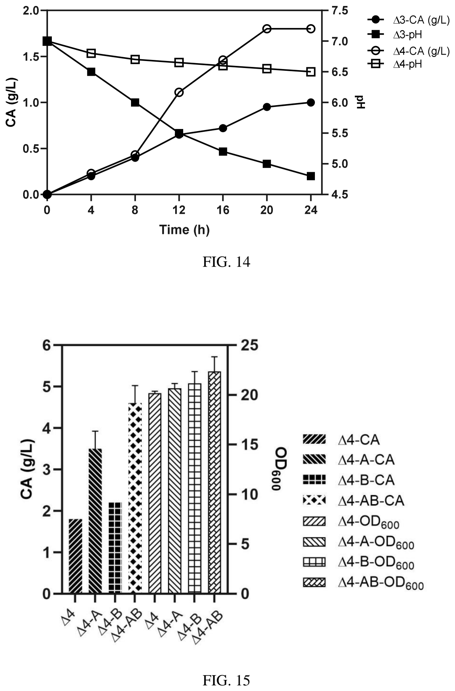

The Δ3 strain constructed in Example 3 was used as a starting strain, and the homologous arm poxB-arm with poxB (gene ID: 946132) knocked out and sgRNA plasmid pTarget-poxB were constructed, respectively, by using the same technical means as in Example 1, to obtain an engineered strain Δ4 with the acetic acid synthesis pathway gene poxB knocked out. Then, the Δ3 and Δ4 strains were analyzed and verified by shake flask, and the corresponding colanic acid production and pH change are shown in FIG. 14. The highest yield of the control strain Δ3 was approximately at the level of 1.0 g/L, while the highest yield of the strain Δ4 with the acetic acid synthesis pathway gene poxB knocked out reached 1.8 g/L; with the extension of the fermentation time, the pH of the fermentation broths corresponding to both strains was continuously reduced, while the pH change of the strain Δ4 was relatively not very significant, reducing from an initial pH7.0 to pH6.5, compared to the control group, reducing from pH7.0 to pH4.8. The results show that the acetic acid synthesis amount can be reduced by blocking the acetic acid synthesis pathway, and since the pH change of the fermentation broth is small, the strain growth environment is more suitable for the growth of strains, thereby further improving the yield of colanic acid.

### Example 13. Integration and Overexpression of rcsA and rcsB at Genome Level

In Example 4, it was found that overexpression of rcsA and/or rcsB by the plasmid would have a significant perturbation effect on the synthesis of colanic acid. Here, the gene rcsA and/or rcsB was integrated into the genome of the engineered strain Δ4 by genomic integration. The operation process proceeded as follows.

### 1. Integration of Homologous Arm Fragment and Construction of sgRNA Plasmid

In order to integrate gene rcsA and/or rcsB into the genome, the corresponding 1000 bp nucleotide sequence on the upstream and downstream of the integration site as well as the gene sequence to be integrated were firstly amplified by PCR, named as rcsA-arm1, rcsA, rcsA-arm2; rcsB-arm1, resB, rcsB-arm2, respectively. When the gene sequences rcsA and rcsB are amplified, a promoter and an RBS sequence were added to the primers. Two corresponding upstream and downstream homologous arms as well as gene sequences were ligated into one fragment by fusion PCR to obtain DNA fragments rcsA-arm and rcsB-arm.

In order to construct the sgRNA, the sgRNA sequence was designed online by using the chopchop website with the corresponding gene sequence as a template, and the obtained sequence was ligated to the pTarget plasmid by PCR to obtain plasmids containing the sgRNAs of the corresponding genes, which were pTarget-rcsA and pTarget-rcsB, respectively.

### 2. Preparation of E. coli Competent Cells

This step is the same as step 2 in Eample 1.

### 3. Electrotransformation Knockout

The obtained DNA fragments rcsA-arm, rcsB-arm and sgRNA pTarget-rcsA, pTarget-rcsB plasmids were added to competent cells consecutively at a ratio of 1:1~1:4, mixed gently, and subjected to ice bath for 30 minutes; the cells were transferred to an electrotransformation cup for electrotransformation (1 mm electrotransformation cup, 1800V, 200 Ω), 600 µL of pre-cooled LB medium was added immediately after electrotransformation, and the cells were transferred to a 1.5 mL EP tube, and cultured at 30°C for 2.5-3 hours. The cells were then plated on spectinomycin- and kanamycin-resistant plates and cultured at 30 °C until colony formation, and then PCR verification of the knockout results was performed

### 4. Elimination of Plasmids

The plasmid elimination step was the same as step 4 in Example 1. Finally, recombinant strains with genomic integration and expression of rcsA, rcsB, and rcsA+rcsB were obtained and named Δ4-A, Δ4-B, and Δ4-AB, respectively. The relevant strains were subjected to shake flask fermentation analysis, and the results are shown in FIG. 15. It can be seen from the results that, when rcsA, rcsB and rcsA+rcsB were enhanced, the yield of colanic acid increased by different degrees, up to 4.6 g/L (Δ4-AB), which was 2.56 times that of the control strain (Δ4). When rcsB was integrated and expressed, the yield reached 2.2 g/L, which was increased by 0.4 g/L compared with the control strain, while in Example 4, the result showed that the yield was reduced by 0.1 g/L when rcsB was overexpressed by the plasmid alone, and the analysis reason may be that when rcsB was overexpressed by the plasmid alone, the expression level was too high, resulting in metabolic balance disorder, thereby having a certain influence on the yield; and when rcsB was overexpressed by integration, since only one copy number was increased due to integration into the genome, the rcsB was not up-regulated too much, and the stability and coordination of the metabolism of the strain were ensured while enhancing the gene, thereby improving the yield of the strain.

### Example 14. Overexpression of NADPH and GTP Cofactor Regeneration Pathway Genes Improving the Synthesis of Colanic Acid

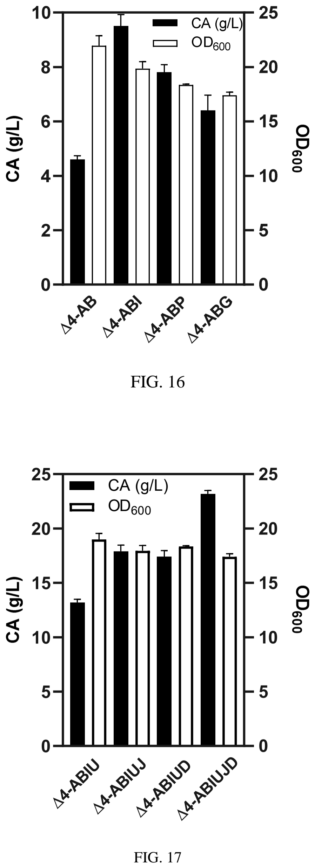

In some enzyme catalysis processes, cofactors such as NADPH and GTP will affect the activity of the enzyme, and in the anabolic process of the colanic acid, many enzymes require cofactors, so the yield of colanic acid can be increased by enhancing the synthesis pathway of the cofactors. By using the operation method described in Example 13, based on strain Δ4-AB, isocitrate dehydrogenase gene icd (gene ID: 945702), pyridine nucleotide transhydrogenase coding gene pntAB (gene ID: 946628, 946144) and guanosine kinase coding gene gsk (gene ID: 946584) were integrated and overexpressed respectively to obtain recombinant strain Δ4-ABI, Δ4-ABP and Δ4-ABG. The relevant strains were subjected to shake flask fermentation analysis, and the results are shown in FIG. 16. It can be seen from the results that, when icd, pntAB and gsk were enhanced, there were positive results, and the yield of colanic acid increased by different degrees, reaching 9.5 g/L (Δ4-ABI), 7.8 g/L (Δ4-ABP) and 6.4 g/L (Δ4-ABG), respectively, and the strain Δ4-ABI with the highest yield improvement was 2.1 times that of the control strain (Δ4-AB). It can be seen from the OD600 that, compared with the control, the strain OD600 was slightly reduced by further enhancing different genes, possibly due to the increase of cofactors which has a certain toxic effect on cells.

### Example 15 Construction of Pathway Gene galU Integrated Expression Strain to Improve the Yield of Colanic Acid

Based on the above strain Δ4-ABI, the pathway key gene galU (gene ID: 945730) was further overexpressed to obtain an engineered strain Δ4-ABIU, and the strain was tested at the shake flask level to have a yield of 13.2 g/L of colanic acid, which was improved by 38% compared with the control strain, and the growth OD600 of the strain was basically similar to that of the control strain.

### Example 16 Construction of Colanic Acid Synthesis Cluster Gene Overexpressed Strain and Improvement of Yield of Colanic Acid

Colanic acid synthesis cluster comprises more than a dozen genes related to colanic acid synthesis that synergistically control colanic acid synthesis. wcaJ (gene ID: 946583) is the first step in the polymerization of colanic acid, transferring UDP-glucose to start the first step synthesis; wcaD (gene ID: 946550) encodes colanic acid polymerase, which polymerizes the basic units into large molecular weight colanic acid. Here, wcaJ, wcaD and wcaJ+wcaD were further overexpressed based on strain Δ4-ABIU to obtain engineered strain Δ4-ABIUJ, Δ4-ABIUD and Δ4-ABIUJD. The relevant strains were subjected to shake flask fermentation analysis, and the results are shown in FIG. 17. It can be seen from the results that, the the yield of colanic acid increased by different degrees, reaching 17.9 g/L (Δ4-ABIUJ), 17.4 g/L (Δ4-ABIUD) and 23.2 g/L (Δ4-ABIUJD), respectively, and the strain Δ4-ABIUJD with the highest yield improvement has increased by 75.7% compared with the control strain (Δ4-ABIU). The growth states of OD600s corresponding to different strains were analyzed to be basically consistent, and overexpression of related genes had no significant effect on the strains.

### Example 17 5-L Fermentation Tank Amplification Verification of Strain Production Performance

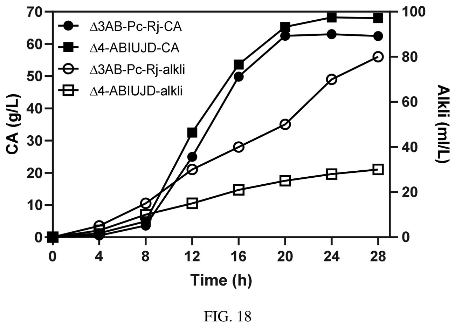

The yield of the optimal production strain Δ3AB-Pc-Rj obtained in Example 11 reached 25.8 g/L in the shake flask level test, and the highest yield of the optimal production strain Δ4-ABIUJD obtained in Example 16 reached 23.2 g/L in the shake flask level test. Here, the two strains were further amplified into a 5-L fermentation tank for comparison and verification of their production performance. The comparison results of strain yields are shown in FIG. 18. The highest yields of strains Δ3AB-Pc-Rj and Δ4-ABIUJD in the 5-L fermentation tank reached 63.0 g/L and 68.2 g/L, respectively, and the two strains reached the highest fermentation at 24 h, which is also the currently reported strain with the highest yield and production efficiency. The supplementation analysis of the alkaline solution shows that the final supplementation amount of the alkaline solution of the strain Δ3AB-Pc-Rj was 80 mL/L, and the supplementation amount of the alkaline solution of the strain Δ4-ABIUJD was 30 mL/L, indicating that the strain Δ4-ABIUJD produced less acid during the fermentation process, which may also be one of the reasons why the yield of this strain is higher than that of the strain Δ3AB-Pc-Rj during the final amplification.

In the case where the starting strain are *E. coli* BL21 (DE3), JM109, Nissle 1917 (EcN), BW23110 or MG1655, respectively, the gene knockout or gene overexpression or optimization of Example 1-3 and Example 12-17 was repeated, and the obtained optimized strain can achieve significant improvement of CA yield.

### INCORPORATION BY RFERENCE

The entire contents of each patent and scientific literature mentioned herein are incorporated herein by reference for all purposes.

### EQUIVALENTS

The present invention may be embodied in other specific forms without departing from its spirit or essential characteristics. The foregoing embodiments are therefore to be considered in all respects illustrative rather than limiting of the invention described herein. The scope of the invention is, therefore, indicated by the appended claims rather than by the foregoing description, and all changes which come within the meaning and scope of equivalency of the claims are intended to be encompassed therein.

## Claims

1. A recombinant engineered strain, wherein in the recombinant engineered strain,
a) lipopolysaccharide core polysaccharide synthesis gene cluster waaL, waaU, waaZ, waaY, waaR, waaO, waaB, waaP, waaG, and waaQ in the genome have been knocked out; and Lon protein coding gene lon and HNS regulatory protein coding gene hns have been knocked out;
b) DNA binding transcription activator RcsA coding gene rcsA and DNA binding transcription activator RcsB coding gene rcsB have been overexpressed;
c) UTP-1-glucose phosphate uridylyltransferase coding gene galU, UDP-glucose-4-isomerase coding gene galE, UDP-glucose-6-dehydrogenase coding gene ugD, phosphomannanase coding gene manB, mannose-1-phosphate guanylyl transferase coding gene manC, GDP-mannose-4,6-dehydratase coding gene gmd, and GDP-L-fucose synthase coding gene fcl have been overexpressed.

2. The recombinant engineered strain of claim 1, wherein the genes overexpressed in b) and c) are derived from any one of *E. coli, Bacillus subtilis, Saccharomyces cerevisiae, Pichia pastoris, Kluyveromyces lactis,* or *Streptomycetaceae,* preferably *E. coli.*

3. The recombinant engineered strain of claim 1, wherein in the recombinant engineered strain, a Ptac promoter is used to enhance the expression of rcsA, rcsB, galU, galE, ugD, manB, manC, gmd and fcl genes.

4. The recombinant engineered strain of claim 3, wherein in the recombinant engineered strain, a Ptac promoter P2 is used to enhance the expression of rcsA and rcsB, a Ptac promoter P3 is used to enhance the expression of galU, galE, and ugD, a Ptac promoter P4 is used to enhance the expression of manB and manC, a Ptac promoter P1 is used to enhance the expression of gmd and fcl, wherein the sequence of said P1 comprises SEQ ID NO: 1, the sequence of said P2 comprises SEQ ID NO: 2, the sequence of said P3 comprises SEQ ID NO: 3, the sequence of said P4 comprises SEQ ID NO: 4.

5. The recombinant engineered strain of any one of claims 1-4, wherein the 5' ends of the rcsA, rcsB, galU, galE, ugD, manB, manC, gmd, and fcl genes are linked to ribosome binding site RBS sequence.

6. The recombinant engineered strain of claim 5, wherein the ribosome binding site RBS sequence comprises a sequence selected from the group consisting of SEQ ID NOs: 5-31.

7. The recombinant engineered strain of any one of claims 1-6, wherein the 5' end of rcsA is linked to a ribosome binding site RBS sequence R_{H1}, the 5' end of rcsB is linked to a ribosome binding site RBS sequence R_{H2}, the 5' end of galU is linked to a ribosome binding site RBS sequence R_{L3}, the 5' end of galE is linked to a ribosome binding site RBS sequence R_{L4}, the 5' end of ugD is linked to a ribosome binding site RBS sequence R_{L5}, the 5' end of manB is linked to a ribosome binding site RBS sequence R_{M6}, the 5' end of manC is linked to a ribosome binding site RBS sequence R_{M7}, the 5' end of gmd is linked to a ribosome binding site RBS sequence R_{L8}, the 5' end of fcl is linked to a ribosome binding site RBS sequence R_{L9}; wherein
the R_{H1} sequence comprises SEQ ID NO: 5;
the R_{H2} sequence comprises SEQ ID NO: 6;
the R_{L3} sequence comprises SEQ ID NO: 25;
the R_{L4} sequence comprises SEQ ID NO: 26;
the R_{L5} sequence comprises SEQ ID NO: 27;
the R_{M6} sequence comprises SEQ ID NO: 19;
the R_{M7} sequence comprises SEQ ID NO: 20;
the R_{L8} sequence comprises SEQ ID NO: 30;
the R_{L9} sequence comprises SEQ ID NO: 31.

8. The recombinant engineered strain of any one of claims 1-7, wherein the recombinant engineered strain is selected from any one of the *Enterobacteriaceae* family, more preferably from *Escherichia coli* BL21 (DE3), JM109, Nissle 1917 (EcN), BW23110 or MG1655.

9. A method for constructing an engineered strain that produces colanic acid, comprising:
a) knocking out lipopolysaccharide core polysaccharide synthesis gene cluster waaL, waaU, waaZ, waaY, waaR, waaO, waaB, waaS, waaP, waaG, waaQ in the genome, and knocking out Lon protein coding gene lon and HNS regulatory protein coding gene hns;
b) overexpressing DNA binding transcription activator RcsA coding gene rcsA and DNA binding transcription activator RcsB coding gene rcsB;
c) overexpressing galU, galE, ugD, manB, manC, gmd and fcl genes.

10. The method of claim 9, wherein the genes overexpressed in b) and c) are derived from any one of *E. coli, Bacillus subtilis, Saccharomyces cerevisiae, Pichia pastoris, Kluyveromyces lactis,* or *Streptomycetaceae,* preferably *E. coli.*

11. The method of claim 9, wherein in the recombinant engineered strain, a Ptac promoter is used to enhance the expression of rcsA, rcsB, galU, galE, ugD, manB, manC, gmd and fcl genes.

12. The method of claim 11, wherein
in the recombinant engineered strain, a Ptac promoter P2 is used to enhance the expression of rcsA and rcsB, a Ptac promoter P3 is used to enhance the expression of galU, galE, and ugD, and a Ptac promoter P4 is used to enhance the expression of manB and manC, a Ptac promoter P1 is used to enhance the expression of gmd and fcl, wherein the sequence of said P1 comprises SEQ ID NO: 1, the sequence of said P2 comprises SEQ ID NO: 2, the sequence of said P3 comprises SEQ ID NO: 3, the sequence of said P4 comprises SEQ ID NO: 4.

13. The method of any one of claims 9-12, wherein
the 5' ends of the rcsA, rcsB, galU, galE, ugD, manB, manC, gmd, and fcl genes are linked to ribosome binding site RBS sequence.

14. The method of any one of claim 13, wherein the ribosome binding site RBS sequence comprises a sequence selected from the group consisting of SEQ ID NOs: 5-31.

15. The method of any one of claim 13 or 14, wherein the 5' end of rcsA is linked to a ribosome binding site RBS sequence R_{H1}, the 5' end of rcsB is linked to a ribosome binding site RBS sequence R_{H2}, the 5' end of galU is linked to a ribosome binding site RBS sequence R_{L3}, the 5' end of galE is linked to a ribosome binding site RBS sequence R_{L4}, the 5' end of ugD is linked to a ribosome binding site RBS sequence R_{L5}, the 5' end of manB is linked to a ribosome binding site RBS sequence R_{M6}, the 5' end of manC is linked to a ribosome binding site RBS sequence R_{M7}, the 5' end of gmd is linked to a ribosome binding site RBS sequence R_{L8}, the 5' end of fcl is linked to a ribosome binding site RBS sequence R_{L9}; wherein
the R_{H1} sequence comprises SEQ ID NO: 5;
the R_{H2} sequence comprises SEQ ID NO: 6;
the R_{L3} sequence comprises SEQ ID NO: 25;
the R_{L4} sequence comprises SEQ ID NO: 26;
the R_{L5} sequence comprises SEQ ID NO: 27;
the R_{M6} sequence comprises SEQ ID NO: 19;
the R_{M7} sequence comprises SEQ ID NO: 20;
the R_{L8} sequence comprises SEQ ID NO: 30;
the R_{L9} sequence comprises SEQ ID NO: 31.

16. The method of any one of claims 9-15, wherein the recombinant engineered strain is selected from any one of the *Enterobacteriaceae* family, more preferably from *Escherichia coli* BL21 (DE3), JM109, Nissle 1917 (EcN), BW23110 or MG1655.

17. Use of the recombinant engineered strain of any one of claims 1-8 in the preparation of a product for producing colanic acid.

18. A recombinant engineered strain, wherein in the recombinant engineered strain,
a) lipopolysaccharide core polysaccharide synthesis gene cluster waaL, waaU, waaZ, waaY, waaR, waaO, waaB, waaP, waaG, and waaQ in the genome have been knocked out; and Lon protein coding gene lon and HNS regulatory protein coding gene hns have been knocked out;
b) acetic acid synthesis pathway gene poxB has been knocked out;
c) DNA binding transcription activator coding gene rcsA and DNA binding transcription activator coding gene rcsB have been overexpressed;
d) isocitrate dehydrogenase gene icd related to NADPH regeneration and/or pyridine nucleotide transhydrogenase coding gene pntAB have been overexpressed; guanosine kinase coding gene gsk related to GTP regeneration has been integrated and expressed;
e) UDP-1-glucose phosphate uridylyltransferase coding gene galU has been overexpressed;
f) UDP-glucose-1-phosphotransferase coding gene wcaJ and/or colanic acid polymerase coding gene wcaD related to colanic acid synthesis cluster have been overexpressed.

19. The recombinant engineered strain of claim 18, wherein the genes overexpressed in c) to f) are derived from any one of *E. coli, Bacillus subtilis, Saccharomyces cerevisiae, Pichia pastoris, Kluyveromyces lactis,* or *Streptomycetaceae,* preferably *E. coli.*

20. A method for contructing a colanic acid recombinant engineered strain, comprising:
a) knocking out lipopolysaccharide core polysaccharide synthesis gene cluster waaL, waaU, waaZ, waaY, waaR, waaO, waaB, waaP, waaG, and waaQ in the genome, and knocking out Lon protein coding gene lon and HNS regulatory protein coding gene hns;
b) knocking out acetic acid synthesis pathway gene poxB;
c) overexpressing DNA binding transcription activator coding gene rcsA and DNA binding transcription activator coding gene rcsB via genomic integration;
d) overexpressing isocitrate dehydrogenase gene icd related to NADPH regeneration and/or pyridine nucleotide transhydrogenase coding gene pntAB; integrating and expressing guanosine kinase coding gene gsk related to GTP regeneration;
e) overexpressing UDP-1-glucose phosphate uridylyltransferase coding gene galU;
f) overexpressing UDP-glucose-1-phosphotransferase coding gene wcaJ and/or colanic acid polymerase coding gene wcaD related to colanic acid synthesis cluster.

21. The recombinant engineered strain of claim 20, wherein the genes overexpressed in c) to f) are derived from any one of *E. coli, Bacillus subtilis, Saccharomyces cerevisiae, Pichia pastoris, Kluyveromyces lactis,* or *Streptomycetaceae,* preferably *E. coli.*
